# DEMANDE DE BREVET EUROPEEN

(11) **EP 0 700 669 A1**
(43) Date de publication de la demande: **13.03.1996**
(21) Numéro de dépôt: 95402065.7
(22) Date de dépôt: 12.09.1995
(51) Int. Cl.: A61F 2/00, A61B 19/00

(54) **Prothèse médicale pour cure d'incontinence urinaire chez la femme**

(30) Priorité: 12.09.1994 FR 9410869
(71) Demandeur: PETERS S.A., F-93000 Bobigny (FR)
(72) Inventeur: Manhes, Hubert, F-03200 Vichy (FR)
(74) Mandataire: Michelet, Alain

(57) **Abrégé**

Prothèse médicale destinée à être implantée dans le vagin (7) pour remédier aux incontinences urinaires; ladite prothèse comprend:
- un corps extensible (15) placé dans le vagin (7) et exerçant une pression constante, d'une part, en arrière sur le cul de sac vaginal postérieur (14) et, d'autre part, en avant sur la symphyse pubienne (3),
- des moyens de gonflage (8) reliés au corps extensible (15) et placés à l'extérieur,
   ledit corps extensible (15) destiné à être placé au cours d'une intervention chirurgicale jusqu'à la cicatrisation sous contrôle celioscopique pour assurer le déplacement et maintien du bloc viscéral utéro-vagino-vésical comprend:
- une partie semi-rigide en périphérie (12),
- une membrane (13) présentant une forme gonflée au centre.

## Description

La présente invention concerne le traitement de l'incontinence urinaire chez la femme par "Retzius Plastie". Plus précisément, elle concerne une prothèse destinée à être placée dans le vagin au cours d'une intervention chirurgicale sous contrôle celioscopique. Cette combinaison de gestes fait que la vessie reste surélevée après le retrait de la prothèse, c'est-à-dire après cicatrisation. La forme de la prothèse est conçue pour surélever deux organes: la vessie et l'utérus. Cette pathologie est fréquente, puisqu'on estime à 20% le nombre des femmes atteintes par cette infirmité.

Dans les traitements connus de l'incontinence urinaire des femmes, il y a actuellement plus de cent techniques chirurgicales traditionnelles différentes qui visent toutes à corriger la position urètre-vessie par suspension à l'aide de fils. Ces techniques supposent toutes un abord chirurgical par laparotomie ou plus récemment pour quelques unes un abord celioscopique. La difficulté rencontrée dans ces techniques est le déplacement des organes pendant la durée de la cicatrisation.

Il est connu dans l'état de la technique:
- Le brevet EP-A-0.498.912, qui décrit un dispositif réglant les problèmes d'incontinence féminine pendant un exercice physique, toux, .... Ce dispositif comprend un anneau cylindrique avec des moyens de gonflage et dégonflage qui est posé à l'intérieur du vagin et supporte le tissu vaginal de chaque côté de l'urètre supérieure. Cet anneau possède deux protubérances en forme de chape nécessaires pour l'introduction dans le vagin.
- Le brevet US-A-4.669.478, qui décrit un dispositif pour le soulagement des problèmes d'incontinence. Le dispositif comporte un élément de forme spéciale, allongée avec des évidements vers le haut. L'élément remplit complètement le vagin et possède un moyen de gonflage.
- Le brevet FR-A-2.698781, qui décrit un corps rigide destiné à être placé dans le vagin pour remédier aux incontinences féminines, ce corps est rigide, ovale et ne possède aucun moyen de gonflage/dégonflage.

Le but de la présente invention est une prothèse destinée à être placée dans le vagin au cours d'une intervention chirurgicale sous contrôle celioscopique. Elle permet d'assurer le déplacement et le maintien des organes: la vessie et l'utérus, pendant l'opération et pendant la durée de la cicatrisation. Cette technique fondamentalement différente de ce qui est connu, fait appel aux propres facultés de réparation de l'organisme. L'espace de Retzius est l'interface entre un plan fixe antérieur, le bloc musculo-aponévrotique et osseux et un plan mobile, le bloc viscéral urétro-vagino-vésical.

L'invention a pour but de:
- Mobiliser l'espace de Retzius de façon à amener le sphincter vésico-urétral à devenir continent en surélevant le plan mobile viscéral, grâce à une prothèse originale positionnée dans le vagin.
- Préserver l'intégrité du Retzius. La technique opératoire intéresse la totalité de la surface de cet espace et s'oppose donc fondamentalement à la technique classique transfixiante de suspension par fils.
- Faire appel au génie réparateur de l'organisme en déclenchant et canalisant une réaction fibroblastique.

Les moyens nécessaires pour cette invention sont les suivants:
. une celioscopie trans-ombilicale, transpéritonéale permettant un bilan abdominal complet. Cette technique peut être réalisée par voie prépéritonéale avec l'instrumentation habituelle,
. de la colle biologique, (par exemple 2 cc de fibrine avec les moyens nécessaires à sa vaporisation),
. un treillis résorbable (par exemple en acide polyglycolique composite type 9: 60% résorbable et 40% non résorbable),
. une prothèse vaginale selon l'invention, extensible ou gonflable: dénommée PRP (prothèse pour Retzius Plastie).

La méthode opératoire est la suivante, elle consiste en:
1. - L'ouverture par celiochirurgie de l'espace de Retzius entre le pubis et la vessie. L'ouverture du péritoine est effectuée par celioscopie à équidistance entre l'ombilic et le dôme vésical. L'incision faite avec l'aiguille monopolaire du palpateur armé est transversale, sectionne l'ouraque et va d'une artère ombilicale à l'autre. Le Retzius est disséqué au palpateur mousse jusqu'au bord inférieur du pubis mettant ainsi à jour l'urètre au milieu prolongé par la vessie en haut et les deux joues du vagin latéralement. Cette dissection est complètement exsangue.
2. - L'encollage de l'espace de Retzius à l'aide d'une colle biologique à résorption semi-rapide et d'un treillis à résorption lente. Le treillis résorbable préalablement découpé à la forme et à la dimension voulue est étalé dans le Retzius. Puis on applique par vaporisation une couche de colle biologique sur ce treillis mais également dans tout l'espace de Retzius.
3. - Un déplacement de l'espace de Retzius. Maintien de l'ensemble en place par la prothèse de la présente invention. La prothèse originale est alors placée dans le vagin prenant appui, en arrière dans le cul de sac vaginal postérieur et, en avant, derrière la symphyse pubienne. La prothèse est gonflée avec 100 à 120 cc d'air sous contrôle celioscopique refoulant la paroi antérieure du vagin contre la symphyse pubienne et remontant ainsi le sphincter urétral. Aucune suture péritonéale n'est effectuée. La brèche se ferme spontanément par accolement des deux feuillets du péritoine sous l'effet combiné de la colle de fibrine et du remplissage de la vessie en fin d'intervention (300 à 500 cc). Cette réplétion est maintenue pendant 20 minutes.

La durée de cette intervention est d'environ 20 minutes. Une sonde urinaire est laissée en place 24 h. La sortie est autorisée à 48 h. Les patientes sont revues au quinzième jour postopératoire pour l'ablation de la prothèse vaginale.

Cette méthode originale s'appuie sur une "philosophie chirurgicale" directement inspirée de l'endoscopie, c'est-à-dire sans le moindre traumatisme, l'exploitation rationnelle des plans de clivage et l'utilisation des facultés de réparation spontanée des tissus. Elle ouvre de nouvelles perspectives thérapeutiques non seulement pour l'incontinence urinaire d'effort mais aussi pour toute chirurgie de prévention et de réparation des lésions de la statique pelvienne.

La prothèse selon l'invention, après avoir été introduite dans le vagin, doit pouvoir y rester sans trop de gène et doit être apte à exercer une pression douce et constante pendant au moins trois jours jusqu'à ce que la colle prenne et que la cicatrisation se fasse.

La prothèse selon l'invention prend appui sur le bas arrière du vagin et exerce comme un coussin d'air une pression vers le haut en avant par déformation de la membrane supérieure élastique vers le haut. La vessie ainsi repositionnée, permet au sphincter vésical de mieux exercer sa fonction d'occlusion. Le gonflement de la prothèse permet ainsi le déplacement et le blocage du bloc viscéral utéro-vagino-vésical. Après cicatrisation d'environ quinze jours, celle-ci est alors retirée.

L'invention concerne une prothèse médicale destinée à être implantée dans le vagin pour remédier aux incontinences urinaires; ladite prothèse comprend:
- un corps extensible, placé dans le vagin et exerçant une pression constante d'une part en arrière sur le cul de sac vaginal postérieur et d'autre part en avant sur la symphyse pubienne,
- des moyens de gonflage reliés au corps extensible et placés à l'extérieur.

Selon l'invention, ledit corps extensible, placé au cours d'une intervention chirurgicale jusqu'à la cicatrisation sous contrôle celioscopique pour assurer le déplacement et le maintien du bloc viscéral utéro-vagino-vésical comprend:
- une partie semi-rigide en périphérie,
- une membrane présentant une forme gonflée au centre.

Selon différents modes de réalisation présentant chacun ses propres avantages:
- l'élément extensible est gonflable,
- l'élément extensible augmente en volume à l'aide d'un ressort interne,
- l'élément extensible comporte un fond plat peu déformable, un anneau semi-rigide périphérique, une membrane supérieure élastique, un tuyau d'arrivée d'air, des moyens de retenue d'air et des moyens d'obturation. La membrane supérieure plus déformable que le fond plat assure un positionnement convenable de la prothèse après mise en place;
- l'élément extensible comporte un fond plat, un anneau semi-rigide périphérique, une membrane supérieure élastique et un ressort interne,
- l'anneau semi-rigide périphérique comporte des plis en accordéon déployable,
- le ressort interne est comprimé avant la mise en place dans le vagin,
- la matière de l'élément extensible est du PVC (polychlorure de vinyle),
- la matière de l'élément extensible est du EVA (éthylène vinyle acétate),
- la matière de l'élément extensible est du PU (polyuréthane),
- la matière de l'élément extensible est en élastomère de silicone,
- la matière de l'élément extensible est en caoutchouc ou, en variante, en latex ou feuille anglaise,
- l'élément extensible est destiné à être gonflé par du gaz tel que de l'air à l'aide du tuyau et des moyens de gonflage;
- l'élément extensible est destiné à être gonflé par un liquide tel que de l'eau.

Plusieurs réalisations selon la présente invention sont donc possibles:
- La première permet une augmentation du volume de la prothèse introduite non par la dilatation d'une membrane souple mais par le déploiement des plis, genre accordéon, après gonflage,
- La deuxième version permet l'augmentation du volume par inversement du bombé de la membrane souple après gonflage.
- La troisième version est obtenue en augmentant le volume d'un élément souple par l'extension d'un ressort interne, qui a été comprimé avant l'introduction de la prothèse dans le vagin.
- La quatrième version comporte deux membranes déployables.

Les dispositions, formes et caractéristiques de la présente invention ressortiront de la description détaillée faite en regard des dessins annexés sur lesquels:

La Figure 1 illustre une vue en coupe du bas ventre avec les organes et la prothèse gonflable dans une position expansée.

Les Figures 2A et 2B illustrent une version gonflable de la prothèse genre accordéon, respectivement à l'état comprimé et à l'état gonflé.

Les Figures 3A et 3B illustrent une version gonflable de la prothèse avec membrane supérieure inversée, respectivement à l'état comprimé et à l'état gonflé.

Les Figures 4A et 4B illustrent une version de la prothèse extensible avec membrane supérieure expansée à l'aide d'un ressort interne, respectivement à l'état comprimé et à l'état gonflé.

Les Figures 5A et 5B illustrent une version de la prothèse extensible avec deux membranes déployables, respectivement à l'état comprimé et à l'état gonflé. La Figure 5C est une vue de dessus de cette réalisation.

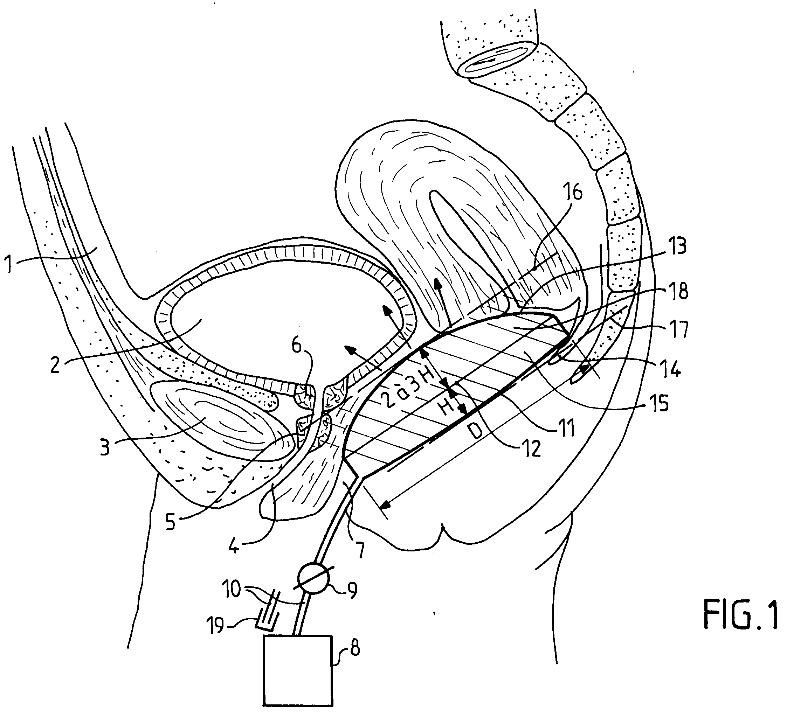

La Figure 1 représente l'élément expansible 15, gonflé et placé dans le vagin 7. L'espace de Retzius 18 est délimité entre le plan fixe antérieur 17 qui est le bloc musculo-aponévrotique et osseux et le plan postérieur mobile 16 qui est le bloc viscéral urétro-vagino-vésical.

On reconnaît sur cette Figure, le péritoine 1, la vessie 2, le sphincter vésico-urétral 5, 6, la symphyse pubienne et l'urètre 4, le cul de sac vaginal postérieur 14.

L'élément expansible 15 est composé d'un fond plat 11 semi-rigide, d'un anneau de renfort 12 de hauteur H également semi-rigide et d'une membrane 13 en matière expansible refoulant la paroi antérieure du vagin 7 contre la symphyse pubienne 3 et remontant ainsi le sphincter urétral 5.

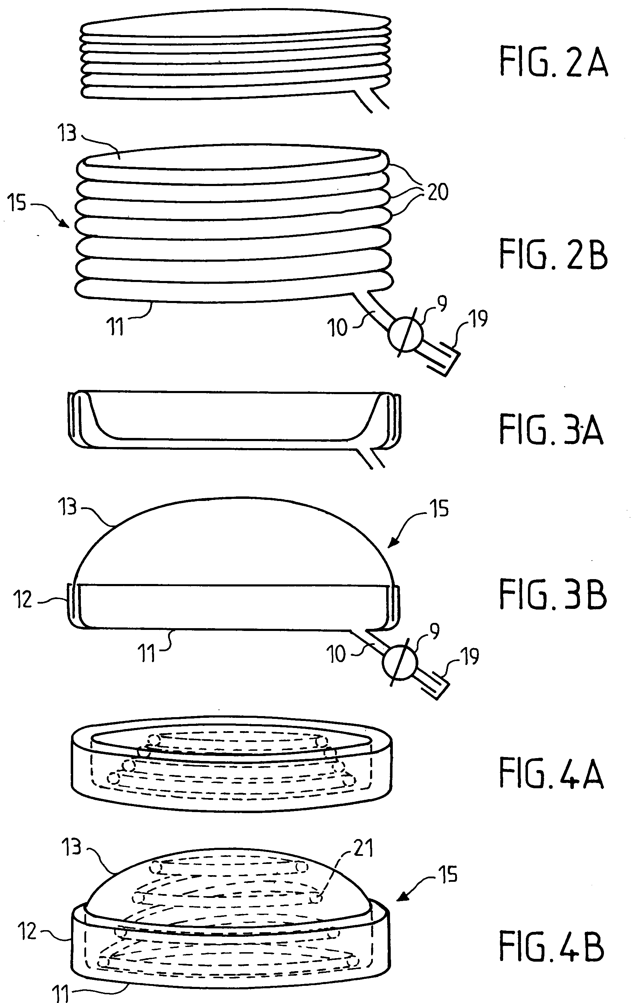

La Figure 2 représente un mode de réalisation de la prothèse 15. Le fond plat 11 est semi-rigide, l'anneau de renfort 12 semi-rigide est remplacé par des plis 20 en accordéon. La membrane supérieure 13 se déplace grâce au déploiement des plis 20 sous l'action de la pression générée par le système de gonflage 8 à travers le tuyau 10. Le clapet anti-retour 9 et l'obturateur 19 permettent le maintien de la pression de l'élément expansible gonflable 15 durant l'opération et la cicatrisation.

La Figure 3 représente un deuxième mode de réalisation de la prothèse comprenant le fond plat 11 semi-rigide, un anneau de renfort 12 semi-rigide et une membrane supérieure 13 en matière expansible semi-inversée, un tuyau 10, un système anti-retour 9 et un obturateur 19. La membrane supérieure 13 se déplace vers le haut sous l'action de la pression générée par le système de gonflage 8 à travers le tuyau 10. Le clapet anti-retour 9 et l'obturateur 19 permettent le maintien de la pression de l'élément expansible gonflable 15 durant l'opération et la cicatrisation.

La Figure 4 représente une troisième réalisation de la prothèse, comprenant le fond plat 11, un anneau semi-rigide 12, une membrane supérieure 13 en matière expansible et un ressort interne 21. L'augmentation de volume de l'élément expansible 15 est assurée par l'extension du ressort interne 21 qui est comprimé avant l'introduction de la prothèse dans le vagin 7 et comprimé à nouveau lors de l'enlèvement de la prothèse le 15e jour.

Dans un mode préférentiel, représenté sur les Figures 3A et 4A, la prothèse présente une partie centrale de la membrane avec une forme gonflée et une partie semi-rigide en périphérie.

Ceci a pour avantage, durant l'intervention chirurgicale sous contrôle celioscopique jusqu'à cicatrisation de combiner l'élévation et le maintien de la vessie et de l'utérus; également après le retrait de la prothèse.

Un autre avantage représenté par exemple dans les Figures 3B et 4B est que l'anneau circulaire semi-rigide permet à la prothèse de rester en place lorsque celle-ci est dégonflée.

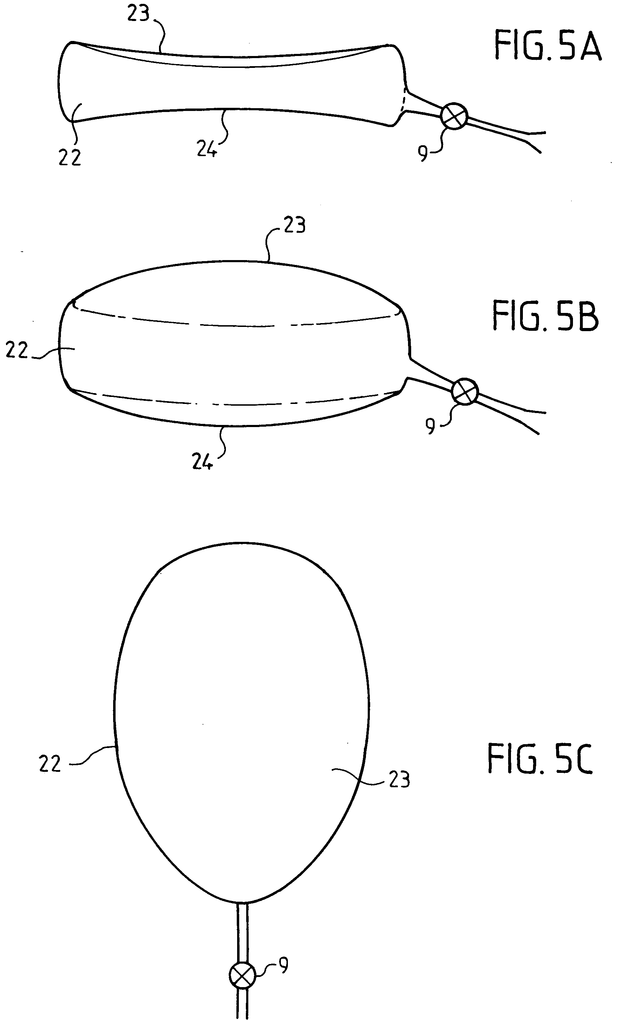

Les Figures 5A, 5B, 5C représentent une quatrième réalisation de la prothèse comprenant un anneau semi-rigide 22, deux membranes supérieure 23 et inférieure 24 gonflables et/ou déployables par un gaz tel que de l'air, ou de l'eau, afin que l'élément double ou triple son volume.

Les formes de l'élément expansible 15 sont rondes ou ovales, d'un diamètre de 8 cm environ. Il est composé d'au moins une membrane dilatable 23, 24 permettant d'augmenter le volume de deux à trois fois en sa partie supérieure. L'élément est obtenu par un moulage, rotomoulage ou soufflage en matière plastique, en PVC, EVA, PU, élastomère de silicone, ou en caoutchouc.

Les signes de référence insérés après les caractéristiques techniques mentionnées dans les revendications, ont pour seul but de faciliter la compréhension de ces dernières, et n'en limitent aucunement la portée.

## Revendications

1. Prothèse médicale destinée à être implantée dans le vagin (7) pour remédier aux incontinences urinaires; ladite prothèse comprend:
- un corps extensible (15) placé dans le vagin (7) et exerçant une pression constante, d'une part, en arrière sur le cul de sac vaginal postérieur (14) et, d'autre part, en avant sur la symphyse pubienne (3),
- des moyens de gonflage (8) reliés au corps extensible (15) et placés à l'extérieur, caractérisé en ce que: ledit corps extensible (15) destiné à être placé au cours d'une intervention chirurgicale jusqu'à la cicatrisation sous contrôle celioscopique pour assurer le déplacement et maintien du bloc viscéral utéro-vagino-vésical comprend:
- une partie semi-rigide en périphérie (12),
- une membrane (13) présentant une forme gonflée au centre.

2. Prothèse médicale selon la revendication 1, caractérisée en ce que l'élément extensible (15) augmente en volume à l'aide d'un ressort interne (21).

3. Prothèse médicale selon l'une des revendications 1 et 2, caractérisée en ce que l'élément extensible comporte un fond plat (11), un anneau semi-rigide périphérique (12), une membrane supérieure élastique (13) se déformant vers le haut.

4. Prothèse médicale selon la revendication 1, caractérisée en ce que l'élément extensible (15) est gonflable par un gaz tel que de l'air, à l'aide du tuyau (10) et des moyens de gonflage (8).

5. Prothèse médicale selon la revendication 4, caractérisée en ce que l'élément extensible comporte un fond plat (11), un anneau semi-rigide périphérique (12), une membrane supérieure élastique (13) se déformant vers le haut, un tuyau d'arrivée d'air (10), des moyens de retenue d'air (9) et des moyens d'obturation (19).

6. Prothèse médicale selon l'une des revendications 4 et 5, caractérisée en ce que l'anneau semi-rigide périphérique (12) comporte des plis (20) en accordéon déployable.

7. Prothèse médicale selon l'une quelconque des revendications 4 à 6, caractérisée en ce que l'élément extensible comporte deux membranes inférieure (23) et supérieure (24) déployables assurant au moins le doublement du volume dudit élément extensible.

8. Prothèse médicale selon la revendication 7, caractérisée en ce que l'élément extensible comporte un anneau semi-rigide (22).

9. Prothèse médicale selon les revendications 4 à 8, caractérisée en ce que l'élément extensible est destiné à être gonflé par un liquide tel que de l'eau.

10. Prothèse médicale selon l'une quelconque des revendications 1 à 9, caractérisée en ce que la matière de l'élément extensible (15) est en PVC.

11. Prothèse médicale selon l'une quelconque des revendications 1 à 9, caractérisée en ce que la matière de l'élément extensible (15) est en EVA.

12. Prothèse médicale selon l'une quelconque des revendications 1 à 9, caractérisée en ce que la matière de l'élément extensible (15) est en PU.

13. Prothèse médicale selon l'une quelconque des revendications 1 à 9, caractérisée en ce que la matière extensible (15) est en élastomère de silicone.

14. Prothèse médicale selon l'une quelconque des revendications 1 à 9, caractérisée en ce que la matière de l'élément extensible (15) est en caoutchouc.
